# EUROPÄISCHE PATENTSCHRIFT

(11) **EP 1 418 875 B1**
(45) Veröffentlichungstag und Bekanntmachung des Hinweises auf die Patenterteilung: **29.03.2006**
(21) Anmeldenummer: 02758264.2
(22) Anmeldetag: 24.06.2002
(51) Int. Cl.: A61F 13/62

(54) **HYGIENEARTIKEL MIT BEFESTIGUNGSELEMENTEN**
HYGIENE ARTICLE WITH FASTENING ELEMENTS
ARTICLE D'HYGIENE POURVU D'ELEMENTS DE FIXATION

(30) Priorität: 18.08.2001 DE 10140622
(43) Veröffentlichungstag der Anmeldung: 19.05.2004
(73) Patentinhaber: Paul Hartmann Aktiengesellschaft, 89522 Heidenheim (DE)
(72) Erfinder: WENDELSTORF, Carsten, 56068 Koblenz (DE); MANGOLD, Rainer, 89542 Herbrechtingen (DE)
(74) Vertreter: Dreiss, Fuhlendorf, Steimle & Becker
(86) Internationale Anmeldenummer: PCT/EP2002/006946
(87) Internationale Veröffentlichungsnummer: WO 2003/015675

(56) Entgegenhaltungen:
- EP-A- 1 151 736
- WO-A-98/22069
- DE-U- 20 005 920
- FR-A- 2 267 058
- GB-A- 1 441 567
- US-A- 4 670 012
- US-A- 5 234 517
- US-A- 6 030 373

## Beschreibung

Die Erfindung betrifft einen Hygieneartikel, insbesondere Windel auch für die Inkontinentenversorgung, mit einer flüssigkeitsdurchlässigen Deckschicht, einer flüssigkeitsundurchlässigen Rückschicht und einem dazwischen angeordneten Saugkörper, und mit Befestigungselementen zum lösbaren Schließen des Hygieneartikels im an einen Benutzer angelegten Zustand, wobei der Hygieneartikel eine erste Längsrichtung und eine zweite Querrichtung aufweist, wobei die Befestigungselemente jeweils ein Fixiermittel tragen und die Fixiermittel mit einem Auftreffbereich des Hygieneartikels zusammenwirken, und wobei das jeweilige Befestigungselement einen ersten Bereich aufweist, mit dem es an den Hygieneartikel angefügt ist, und einen zweiten Bereich, der in Querrichtung von dem ersten Bereich beabstandet angeordnet ist und in dem jeweils das Fixiermittel vorgesehen ist, wobei das jeweilige Befestigungselement mit in der Längsrichtung verlaufenden Falzlinien z-förmig gefaltet ist,

Hygieneartikel mit vor dem Gebrauch auf sich selbst gefalteten Befestigungselementen sind beispielsweise aus EP 0 669 121 A1 oder WO 95/16425 bekannt. Z-förmig auf sich selbst gefaltete Befestigungselements für wegwerfbare Hygieneartikel sind auch aus US 4,237,890 bekannt.

Hiervon ausgehend liegt der vorliegenden Erfindung die Aufgabe zugrunde, einen Hygieneartikel der genannten Art im Hinblick auf die Anbringbarkeit der Befestigungselement bei der Herstellung des Hygieneartikels zu verbessern, aber auch die Handhabung des Hygieneartikels nach dem Anfügen der Befestigungselement in einer schnell laufenden Herstellungsmaschine und während eines anschließenden Verpackungsprozesses zu vereinfachen. Ferner soll mit der vorliegenden Erfindung die Handhabbarkeit des Hygieneartikels beim Entfalten, Positionieren und Schließen der Befestigungselemente im an einen Benutzer angelegten Zustand des Hygieneartikels verbessert werden.

Diese Aufgabe wird bei einem Hygieneartikel der beschriebenen Art erfindungsgemäß dadurch gelöst, dass der das Fixiermittel aufweisende zweite Bereich in Querrichtung außerhalb der z-förmig gefalteten Konfiguration liegt.

Mit der vorliegenden Erfindung wird also vorgeschlagen, das Befestigungselement in der Längsrichtung derart zu falten, dass sich eine Z-förmige Konfiguration ergibt, von der ein Teil oder Abschnitt des Befestigungselements, welcher den zweiten Bereich mit dem Fixiermittel aufweist, seitlich vorsteht, sich also außerhalb der Z-förmig gefalteten Konfiguration befindet. Auf diese Weise ist es möglich, das jeweilige Befestigungselement mit seinem ersten Bereich auf einer Oberfläche des Hygieneartikels festzulegen, d. h. dort anzufügen. Der seitlich, also in Querrichtung, von der Z-förmig gefalteten Konfiguration vorstehende Teil oder Abschnitt des Befestigungselements kann nun erfindungsgemäß leicht gegriffen werden. Da dort das klebend oder in sonstiger Weise haftend ausgebildete Fixiermittel vorgesehen ist, ergeben sich auch Vorteile im Hinblick auf die Fertigung; insbesondere wird vermieden, dass das Fixiermittel in störender Weise mit der z-förmig gefalteten Konfiguration interferiert.

In weiterer Ausbildung der Erfindung ist vorgesehen, dass das jeweilige Befestigungselement mit einem außerhalb der z-förmig gefalteten Konfiguration liegenden Bereicht mit einer Oberseite des Hygieneartikels eine lösbare Halteverbindung eingeht, indem dort beliebige Haft- oder Haltemittel vorgesehen sind. Beispielsweise könnte der zweite Abschnitt des Befestigungselements an irgendeiner Stelle Klebepunkte aufweisen, die an der Oberseite des Hygieneartikels leicht lösbar anhaften und so das Befestigungselement in der bestimmungsgemäßen Montageposition halten. Es wäre auch denkbar, dass der zweite Bereich, welcher das wirkenden Fixiermittel aufweist, von einer ablösbaren Abdeckung überfangen ist, die ihrerseits eine Haftbeschichtung zum lösbaren Halten des Abschnitts an der Oberseite des Hygieneartikels aufweist.

In Weiterbildung der Erfindung wird jedoch vorgeschlagen, dass die lösbare Halteverbindung durch das Fixiermittel im zweiten Bereich des Befestigungselements selbst gebildet wird.

Es erweist sich auch als vorteilhaft, wenn das jeweilige Fixiermittel durch mechanisch wirkende Verschlußelemente gebildet ist. Zur Ausbildung einer löbaren Halteverbindung können diese mit einer textil anmutenden Oberseite des Hygieneartikels beispielsweise nach Art eines Haken/Flausch-Materials lösbar haftend zusammenwirken.

Im Hinblick auf die Anfügung der Befestigungselemente an den Hygieneartikel käme eine Anordnung auf der einem Benutzer zugewandten Innenseite, also auf der Seite der flüssigkeitsdurchlässigen Deckschicht, in Frage, oder auch eine Anbringung über gabel- oder Y-förmige Haltelaschen, welche den Verbund aus Deckschicht und Rückschicht des Hygieneartikels von beiden Seiten umfassen und dort angefügt sind. Es erweist sich hingegen unter Ausführung der vorliegenden Erfindung als besonders vorteilhaft, wenn die Befestigungselemente an eine körperabgewandte Seite des Hygieneartikels angefügt sind.

Die körperabgewandte Seite kann in vorteilhafter Weise eine textil anmutende Oberfläche aufweisen. Diese textil anmutende Oberfläche umfaßt vorteilhafterweise die flüssigkeitsundurchlässige Rückschicht und kann von einem Folien/Vlies-Laminat gebildet sein. Bei dieser Ausführungsform kann die lösbare Halteverbindung beispielsweise dadurch bewirkt werden, dass mechanisch wirkende Verrschlußelemente, die außerhalb der Z-förmig gefalteten Konfiguration angeordnet sind, direkt mit der textil anmutenden Oberfläche haftend zusammenwirken.

Nach einer weiteren besonders bevorzugten Ausführungsform der Erfindung sind die Z-förmig gefalteten Befestigungselemente in Querrichtung derart von einem Längsrand des Hygieneartikels beabstandet, dass der zweite Bereich mit dem Fixiermittel wenigstens nahezu vollständig innerhalb des Längsrands angeordnet ist. Hiermit soll zum Ausdruck gebracht werden, dass der zweite Bereich mit dem Fixiermittel möglichst wenig, vorzugsweise nicht über den Längsrand des Hygieneartikels vorstehen soll. Damit soll verhindert werden, dass beim Einfalten des Hygieneartikels auf die körperzugewandte Deckschicht der überstehenden Bereich des Fixiermittel an der Deckschicht hängenbleibt und diese beim Ablösen beschädigt. Der zweite Bereich sollte nicht mehr als 5 mm, vorzugsweise weniger als 3 mm,, insbesondere weniger als 2 mm in Querrichtung über den Längsrand nach außen überstehen. Bei Einfaltung der Seitenabschnitte des Rückbereichs des Hygieneartikels, die häufig auch als "Ohren" bezeichnet werden und die Befestigungselemente tragen, auf die körperzugewandte Deckschicht im Zuge der Faltung des Hygieneartikels im Herstellungsprozess wird nämlich dann verhindert, dass das jeweilige Fixiermittel sich mit der Oberfläche der meist flauschig ausgebildeten Deckschicht verhakt und dieses beschädiget. Ferner könnte hierbei flauschiges Material an den Fixiermitteln hängenbleiben und die Schließfähigkeit der mechanisch wirkenden Fixiermittel beeinträchtigen.

In noch weiterer Ausbildung der Erfindung wird vorgeschlagen, die Befestigungselemente so auszubilden, dass in Querrichtung außerhalb des zweiten Bereichs des jeweiligen Befestigungselements und auf der von der z-förmig gefalteten Konfiguration abgewandten Seite des zweiten Bereichs ein zum Ergreifen mit einem Finger des Benutzers dienender Anfaßbereich vorgesehen ist. In noch weiterer Ausbildung dieses Erfindungsgedankens ist das Befestigungselement so an der körperabgewandten Oberfläche des Hygieneartikels positioniert und dort angefügt, dass sein Anfaßbereich in Querrichtung über einen Längsrand des Hygieneartikels übersteht. Dies erweist sich insofern als vorteilhaft, als bei ausgebreitetem und ausgefaltetem Hygieneartikel das jeweilige Befestigungselement mit seinem Anfaßbereich in Querrichtung über die beidseiten Längsränder des Hygieneartikels vorstehen und damit sichtbar sind. Sie sind dann aber auch leicht mit den Fingern eines Benutzers greifbar, der mit der einen Hand den Anfaßbereich ergreift und mit der anderen Hand, etwa mit Daumen und Zeigefinger, einen Längsrandbereich des Hygieneartikels ergreift und dann eine Zugbewegung ausübt. Hierbei löst sich die lösbare Halteverbindung zwischen Befestigungselement und Windeloberfläche und die Z-förmig gefaltete Konfiguration wird entfaltet, so dass das Befestigungselement in Hüftumfangsrichtung in Richtung auf den im Vorderbereich des Hygieneartikels vorgesehenen Auftreffbereich bewegt werden kann, um dort zum Schließen Hygieneartikels im an einen Benutzer angelegten Zustand fixiert zu werden.

Das jeweilige Befestigungselement kann einen ersten Abschnitt einer ersten Trägerschicht und einen zweiten Abschnitt einer zweiten Trägerschicht aufweisen, der in Querrichtung neben dem ersten Abschnitt angeordnet und mit diesem verbunden ist. Der erste Abschnitt weist den ersten Bereich und der zweite Abschnitt weist den zweiten Bereich auf.

Es erweist sich dann als vorteilhaft, wenn eine in der Längsrichtung verlaufende Falzlinie des Befestigungselements wenigstens abschnittsweise entlang eines Materialübergangs zwischen erstem und zweitem Abschnitt des Befestigungselements verläuft. Hierdurch wird die Faltbarkeit des Befestigungselements verbessert und die Dicke der Z-förmig gefalteten Konfiguration wird so gering wie möglich gehalten.

Vorzugsweise verläuft die Falzlinie unmittelbar entlang eines Längsrands des zweiten Abschnitts des Befestigungselements.

Der erste und der zweite Abschnitt des jeweiligen Befestigungselements können in an sich beliebiger Weise zusammengefügt sein, sie könnten auf Stoß aneinander anliegen und von einem dritten, insbesondere streifenförmigen Befestigungselement überfangen sein, welches die beiden Abschnitte unlösbar zusammenfügt. Es ist aber auch denkbar und vorteilhaft, dass der erste und der zweite Abschnitt des Befestigungselements in Querrichtung überlappt sind und im Überlappungsbereich zusammengefügt sind. Solchenfalls erweist es sich als vorteilhaft, wenn eine in der Längsrichtung verlaufende Falzlinie des Befestigungselements unmittelbar entlang eines Längsrands des ersten Bereichs verläuft.

In noch weiterer Ausbildung der Erfindung erweist es sich als vorteilhaft, wenn eine weitere Falzlinie der Z-förmig gefalteten Konfiguration unmittelbar entlang eines Längsrands einer Kleberbeschichtung des ersten Bereichs verläuft. Die Kleberbeschichtung des ersten Bereichs kann dabei eine an sich beliebige flächenhafte Erstreckung aufweisen, wobei sich zur Erreichung einer sehr guten Fixierung der Z-förmig gefalteten Konfiguration auf der Oberseite des Hygieneartikels ein möglichst flächenhafter, insbesondere in der Draufsicht rechteckförmig begrenzter Kleberauftrag, als empfehlenswert erweist.

Es erweist sich des weiteren als vorteilhaft, wenn die zweite Trägerschicht des Befestigungselements ein im wesentlichen unelastisches Material umfaßt und in Querrichtung im wesentlichen nicht dehnbar ausgebildet ist. Hingegen wird vorgeschlagen, dass die erste Trägerschicht des Befestigungselements ein zumindest in Querrichtung elastisch dehnbares Material umfaßt und in dieser Querrichtung auch elastisch dehnbar ausgebildet ist.

Unter elastisch dehnbar wird hierunter ein Material verstanden, welches bei Anwendung von Zugkräften zumindest um das 1,2-Fache seiner Ursprungsabmessung gelängt werden kann und sich nach Wegnahme der Zugkräfte zumindest so weit wieder zusammenzieht, dass zumindest die Hälfte der Längung wieder rückgängig gemacht wird. Es versteht sich, dass Materialien bekannt und für die Verwendung als erste Trägerschicht bevorzugt sind, die ein noch viel weitergehenderes elastisches Retraktionsverhalten zeigen.

Zur Erreichung dieser elastisch dehnbaren Eigenschaften der ersten Trägerschicht können in vorteilhafter Weise elastische Vliesmaterialien oder auch sogenannte "Stretchbond"-Laminate mit einer oder mehreren Vlies- und/oder Folienschichten verwendet werden.

In Weiterbildung des Erfindungsgedankens wird vorgeschlagen, auch die Z-förmig gefaltete Konfiguration des Befestigungselements auf sich selbst lösbar zu fixiern. Dies kann beispielsweise dadurch erreicht werden, dass die Z-förmig gefaltete Konfiguration durch eine Anzahl von 1 bis 10, vorzugsweise 3 bis 5 diskreten Fixierpunkten, etwa Schweißpunkten, mit einer Querschnittsfläche von jeweils weniger als 1 mm² fixiert werden, so dass ein Auffalten der Z-förmig gefalteten Konfiguration während des Herstellungs- oder Verpackungsprozesses oder vor dem bestimmungsgemäßen Entfalten beim Anlegen des Hygieneartikels verhindert wird.

Es wurde in Weiterbildung dieses Erfindungsgedankens festgestellt, dass sich eine Fixierung der Z-förmig gefalteten Konfiguration auf sich selbst auch dadurch erreichen läßt, dass beim Abtrennen von ein jeweiliges Befestigungselement bildenden Längsabschnitten in Querrichtung von einer endlosen Bahn am hierbei gebildeten Schnittrand Fasern der Z-förmig übereinander gefalteten Lagen in die darunter angeordnete Lage hineingewirkt oder hineingezogen werden. Es kann also beim Abtrennen dieser Längsabschnitte eine Art Vernadelungseffekt von Faservliesen erreicht werden, der hinreichend ist, um die Z-förmig gefaltete Konfiguration auf sich selbst zu halten. Beim Abtrennen der Längsabschnitte werden insbesondere durch Ausführung eines "Quetschschnittes" Fasern einer Lage in die in Schnittrichtung darunter angeordnete Lage hineingezogen. Dieser "Quetschschnitt" wird vorzugsweise durch ein nachgiebiges, insbesondere unter Vorspannung stehendes, oder zumindest geringfügig nachgiebig gelagertes Schneidmesser ausgeführt. Dieses Schneidmesser ist vorzugsweise auf einer rotierenden Walze angeordnet und beim Ausführen des Schnitts gegen eine Gegendruckwalze unter entsprechendem Druck anlegbar, über welche die Bahn geführt ist und die ein Widerlager für das Schneidmesser bildet.

Nach einem weiteren Erfindungsgedanken von an sich eigenständiger Bedeutung ist in einem Längsendbereich des Rückbereichs beidseits in Querrichtung außen eine Materialaussparung vorgesehen, derart, dass die Erstreckung des Rückbereichs in der Längsrichtung am jeweiligen Längsseitenrand der Windel geringer ist als im Bereich zwischen den Aussparungen und dass die Verschlußelemente und Gegenverschlußelemente so bezüglich dem Rückbereich und dem Vorderbereich angeordnet sind, dass im angelegten Zustand der Windel der Rückbereich sich weiter nach oben erstreckt als der Vorderbereich, der Rückbereich also den Vorderbereich nach oben hin überragt. Mit anderen Worten ausgedrückt bedeutet dies, dass die Fixiermittel im Rückbereich und der Auftreffbereich im Vorderbereich der Windel einen unterschiedlichen Abstand vom zugehörigen Längsende der Windel im Rückbereich bzw. im Vorderbereich haben: Die Fixiermittel sind in Längsrichtung weiter vom Längsende des Rückbereichs der Windel entfernt als der Auftreffbereich vom Längsende des Vorderbereichs.

Hierdurch entsteht der Eindruck eines schlanken Produkts, welches schmal baut und lang ausgebildet ist. Tatsächlich kann hierdurch auch eine größere Längserstreckung der Windel erreicht werden, so z.B. wenn in vorteilhafter Weise die Erstreckung des Rückbereichs in der Längsrichtung größer ist als die Erstreckung des Vorderbereichs.

Die erwähnten Aussparungen sind vorzugsweise als durch die Windel durchgehende Ausstanzungen oder Ausschnitte aus einem die Deckschicht und die Rückschicht umfassenden Laminat gebildet.

In weiterer Ausbildung dieses Erfindungsgedankens ist ein die jeweiligen Aussparungen begrenzender Rand des Rückbereichs im Übergang zu einem Längsseitenrand der Windel auf gleicher Höhe wie ein den Vorderbereich begrenzender Querrand angeordnet.

Betrachtet man die so weitergebildete Windel im an einen Benutzer angelegten Zustand, so ist der Übergang des die Aussparung begrenzenden Randes zum Längsseitenrand der Windel, also der Eck- oder Übergangspunkt des Windelseitenbereichs (auch häufig als Ohrbereich bezeichnet), im wesentlichen auf derselben Höhe angeordnet wie der entsprechende Eck- oder Übergangspunkt des Vorderbereichs.

In weiterer Ausbildung dieses Erfindungsgedankens wird vorgeschlagen, den die Fixiermittel aufweisenden Bereich der Befestigungselemente und den korrespondierenden Auftreffbereich mit einer im wesentlichen einander entsprechenden Längserstreckung auszubilden, so dass der Benutzer hierdurch zu einer korrekten Positionierung der Befestigungsmittel auf dem Auftreffbereich gezwungen wird. Hierdurch wird dann aber eine korrekte Paßform und ein korrektes Anlegen der Windel an den Benutzer erreicht.

Es erweist sich des weiteren als optisch ansprechend und im Hinblick auf die Erreichung einer optimalen Paßform als vorteilhaft, wenn im angelegten Zustand der Windel der die jeweilige Aussparung begrenzende Rand des Rückbereichs so in der Querrichtung ausläuft, dass er stetig in den den Vorderbereich begrenzenden Querrand übergeht oder in diesen ausläuft. Während zur Erreichung des erfindungsgemäßen Effekts auch ein schräg zur Längs- und Querrichtung verlaufender Abschnitt oder Ausschnitt zur Bildung der Aussparungen denkbar wäre, erweist sich die vorstehende Ausführungsform aber als vorteilhaft, da hierdurch ein harmonischer Übergang des die jeweilige Aussparung begrenzenden Rands und des den Vorderbereich begrenzenden Querrands erreicht wird.

In weiterer Ausbildung der Erfindung kann auch im Vorderbereich in einem Längsendbereich beidseits in Querrichtung außen eine zweite Materialaussparung vorgesehen sein, derart, dass die Erstreckung des Vorderbereichs in der Längsrichtung am jeweiligen Längsseitenrand geringer ist als im Bereich zwischen den Aussparungen. Die entsprechende Differenz der Erstreckung wird aber vorzugsweise sehr viel geringer als im Rückbereich sein.

Des weiteren erweist es sich als besonders vorteilhaft, wenn im Rückbereich Befestigungselemente verwendet werden, die eine hinreichende Erstreckung in Längsrichtung von 4 bis 10 cm aufweisen, damit die beim Schließen der Windel auftretenden Zugkräfte gleichmäßig in den mit verhältnismäßig großer Längserstreckung ausgebildeten Rückbereich der Windel eingeleitet werden.

Weitere Einzelheiten und Vorteile der Erfindung ergeben sich aus den beigefügten Patentansprüchen und aus der zeichnerischen Darstellung und nachfolgenden Beschreibung einer bevorzugten Ausführungsform der Erfindung. In dieser Zeichnung zeigt:
- Figur 1: eine Schnittansicht eines Befestigungselements für einen erfindungsgemäßen Hygieneartikel;
- Figur 2: eine Draufsicht auf das Befestigungselement nach Figur 1;
- Figur 3: eine Schnittansicht des Befestigungselements nach Figuren 1 und 2 in Z-förmig gefalteter Konfiguration;
- Figur 4: eine Draufsicht auf einen erfindungsgemäßen Hygieneartikel mit einerseits eingefalteten und andererseits ausgefalteten Befestigungselementen;
- Figur 5: eine photographische Darstellung des im Auflichtmikroskop betrachteten Schnittrands des Z-förmig gefalteten Befestigungselements;
- Figur 6: eine schematische Darstellung einer Schneidvorrichtung zur Vereinzelung von Z-förmig gefalteten Befestigungselementen und
- Figur 7: eine Seitenansicht der erfindungsgemäßen Windel im angelegten Zustand.

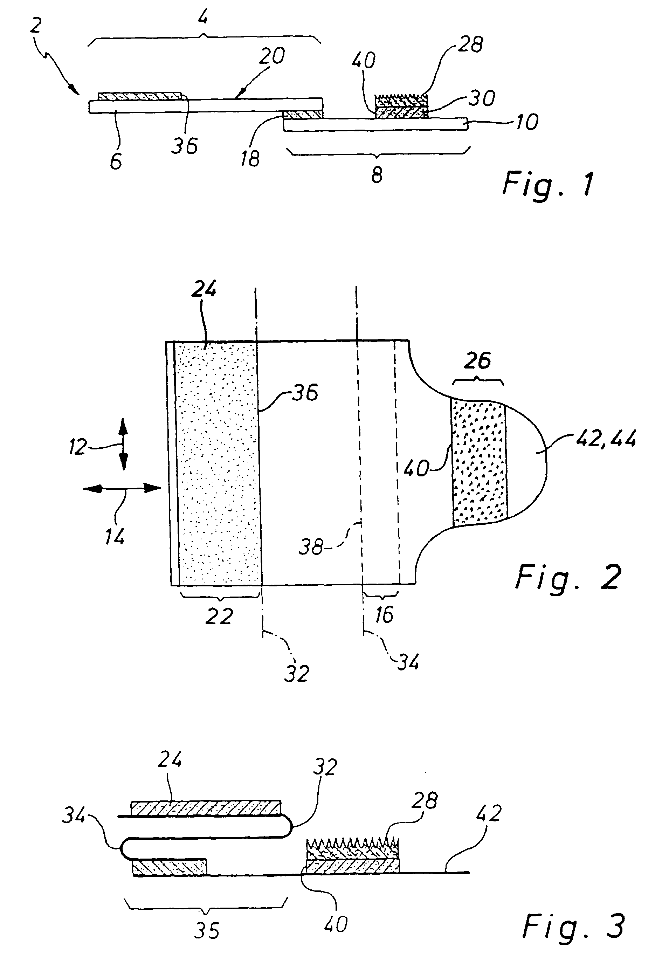

Die Figuren 1 und 2 zeigen ein erfindungsgemäßes Befestigungselement, welches insgesamt mit dem Bezugszeichen 2 bezeichnet ist. Das Befestigungselement 2 umfaßt einen ersten Abschnitt 4 einer ersten Trägerschicht 6 und einen zweiten Abschnitt 8 einer zweiten Trägerschicht 10.

Das Befestigungselement 2 ist als Längsabschnitt von einer endlosen Bahn abgetrennt worden, wobei sich die endlose Bahn in einer ersten Längsrichtung 12 erstreckt. Der zweite Abschnitt 8 ist in einer Querrichtung 14 neben dem ersten Abschnitt 4 angeordnet, wobei im dargestellten Fall der erste Abschnitt 4 und der zweite Abschnitt 8 einander überlappen, so dass ein Überlappungsbereich 16 gebildet ist, an dem die beiden Abschnitte 4, 8 über einen Kleber 18, über Schweißstellen oder in sonstiger Weise unlösbar miteinander verbunden sind.

Auf einer ersten Oberseite 20 des ersten Abschnitts 4 ist ein erster Bereich 22 vorgesehen, der eine Kleberbeschichtung 24 trägt, mit dem das Befestigungselement an einen Hygieneartikel angefügt werden kann.

In einem zweiten Bereich 26 des zweiten Abschnitts 8 sind Fixiermittel 27 in Form von mechanisch wirkenden Verschlußelementen 28, vorzugsweise in Form einer hakenbildenden Komponente eines Haken/Schlaufen-Materials, vorgesehen, insbesondere mittels eines Klebers 30 aufgeklebt. Der zweite Bereich 26 ist vorzugsweise auf derselben Oberseite 20 des Befestigungselements 2 vorgesehen wie der erste Bereich 22.

Des weiteren sind erste und zweite Falzlinien 32, 34 in Figur 2 angedeutet und aus Figur 3 ersichtlich, um welche das Befestigungselement 2 in Längsrichtung 12 Z-förmig gefaltet ist, so dass die in Figur 3 dargestellte Konfiguration 35 gebildet wird. In vorteilhafter Weise verläuft die erste Falzlinie 32 unmittelbar entlang eines Längsrands 36 der Kleberbeschichtung 24 im ersten Bereich 22. Die zweite Falzlinie 34 läuft vorteilhafterweise unmittelbar entlang des Materialübergangs zwischen erstem und zweitem Abschnitt 4 bzw. 8, also entlang eines Rands 38 des Überlappungsbereichs 16.

Wie aus Figur 3 ersichtlich ist der zweite Bereich 26 des zweiten Abschnitts 8 in Querrichtung 14 derart weit vom Überlappungsbereich 16 oder von einem sonstigen Materialübergangsbereich zwischen erstem Abschnitt 4 und zweitem Abschnitt 8 beabstandet, dass er sich in Querrichtung 14 außerhalb der Z-förmig gefalteten Konfiguration 35 des Befestigungselements 2 befindet, was sich in mehrfacher Hinsicht als vorteilhaft erweisen kann. Im dargestellten Fall reicht ein dem ersten Abschnitt 4 zugewandter Längsrand 40 des zweiten Bereichs 26 und damit der mechanisch wirkenden Verschlußelemente 28 in Querrichtung 14 nahezu an die erste Falzlinie 36 bei Z-förmig gefalteter Konfiguration 25 heran. Es wäre aber auch denkbar, dass der zweite Bereich 26 so bezüglich des zweiten Abschnitts 8 positioniert ist, dass die mechanisch wirkenden Verschlußelemente 28 bzw. deren Längsrand 40 einen Abstand von einigen Millimetern von der Z-förmig gefalteten Konfiguration 35 des Befestigungselements aufweisen.

Es sei noch erwähnt, dass der erste Abschnitt 4 in Querrichtung 14 elastisch dehnbar ausgebildet ist und dass der zweite Abschnitt 8 in Querrichtung 14 im wesentlichen nicht dehnbar ausgebildet ist.

Eine Kleberbeschichtung des ersten Bereichs 24 sowie das Fixiermittel 27 im zweiten Bereich 26 können in Längsrichtung 12 durchgehend und endlos auf eine dementsprechend endlose Bahn der ersten Trägerschicht 6 und der zweiten Trägerschicht 8 aufgebracht werden. In entsprechender Weise werden die erste und die zweite Trägerschicht 6, 8 durch einen endlosen Kleberstreifen 18 oder in sonstiger Weise in Längsrichtung 12 kontinuierlich miteinander verbunden. Eine Simultanfertigung zweier spiegelbildlich jedoch in Längsrichtung um n/2 versetzt, wie dies beispielsweise aus EP 0 669 121 A1 bekannt ist, ist ebenfalls denkbar und vorteilhaft.

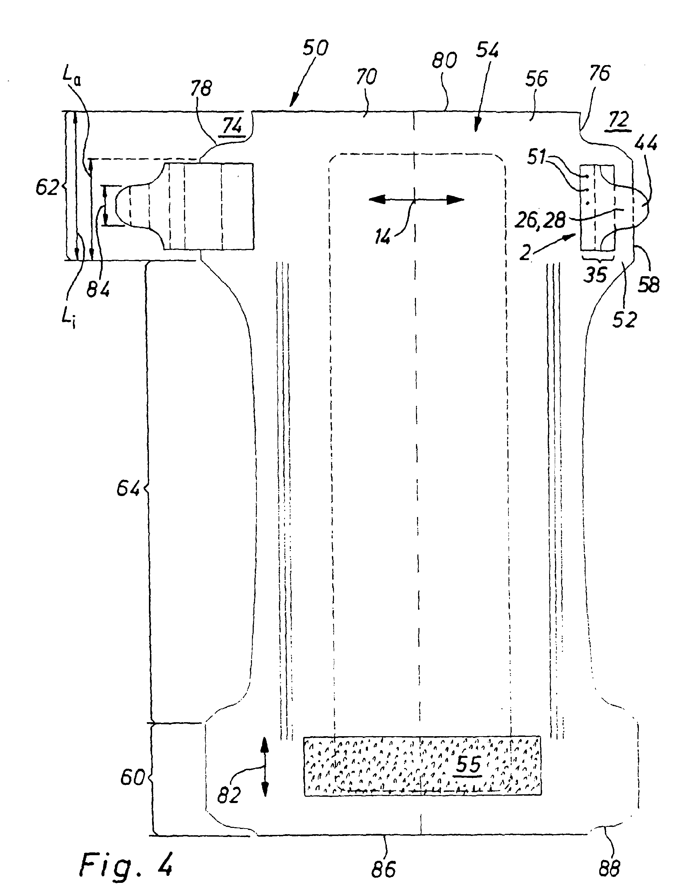

Wenn das Befestigungselement mit seinem Haftkleberauftrag 24 im ersten Bereich 22 auf einen Hygieneartikel aufgebracht ist, so ist - wie bereits erwähnt - der zweite Bereich 26 mit den mechanisch wirkenden Fixiermittelen 28 außerhalb der Z-förmig gefalteten Konfiguration 35 angeordnet und kann somit zum Festlegen des Befestigungselements bzw. des zweiten Abschnitts 8 auf einer textilen Oberfläche eines Hygieneartikels dienen. Dies wird nachfolgend unter Bezugsnahme aus Figur 4 erläutert.

Dieses Festlegen des zweiten Abschnitts 8 an dem Hygieneartikel soll aber lediglich der Fixierung des Befestigungselements während der Herstellung und Verpackung bis zum Anlegen des Hygieneartikels an einen Benutzer dienen, zu welchem Zeitpunkt spätestens diese Verbindung gelöst wird. Ein Benutzer greift dann mit seinen Fingern zwischen die Oberseite des Hygieneartikels und einen freien Endbereich 42 des zweiten Abschnitts 8, der dann als Anfaßbereich 44 dient.

Figur 4 zeigt eine Draufsicht auf eine erfindungsgemäße Windel 50 mit in einem Rückbereich der Windel 50 beidseits angeordneten Befestigungselementen 2, wie sie im Zusammenhang mit den Figuren 1 bis 3 beschrieben wurden. Auf der in Figur 4 linken Seite ist das Befestigungselement 2 entfaltet und auf der rechten Seite ist es Z-förmig gefaltet dargestellt. Das jeweilige Befestigungselement 2 ist mit seinem ersten Bereich 22 und der dort vorgesehenen rechteckförmigen Kleberbeschichtung 24 auf eine Windelaußenseite, d. h. auf eine beim bestimmungsgemäßen Gebrauch abgewandte Oberseite 54 einer flüssigkeitsundurchlässigen Rückschicht 56 der Windel unlösbar aufgebracht. Die Oberseite 54 ist von einer textil anmutenden Vliesbeschichtung einer flüssigkeitsundurchlässigen Folie gebildet. Die Rückschicht 56 ist demzufolge von einem Vlies/Folien-Laminat gebildet.

Das Befestigungselement 2 ist in Querrichtung 14 in einem solchen Abstand von einem Längsrand 58 der Windel 50 in dem Seitenklappen- oder Ohrbereich der Windel 50 angeordnet, dass sie nur mit ihrem Anfaßbereich 44 über den Längsrand 50 in Querrichtung 14 vorstehen. Im Besonderen ist dafür Sorge getragen, dass der zweite Bereich 26, in dem die mechanisch wirkenden Verschlusselemente 28 vorgesehen sind, (in Z-förmiger Faltung des Befestigungselements) nicht oder nur sehr wenig, höchstens 5 mm, über den Längsrand 58 in Querrichtung 14 vorstehen. Hierdurch wird verhindert, dass die Verschlusselemente 28 beim Einschlagen der Seitenklappen- oder Ohrbereiche auf die körperzugewandte Deckschichtlage dort festhaken und damit verbunden Beschädigungen auftreten.

Wie aus Figur 4 im Zusammenhang mit Figur 3 ersichtlich ist, befindet sich der zweite Bereich 26 mit den Verschlusselementen 28 außerhalb der Z-förmig gefalteten Konfiguration 35 und liegt somit gegen die Oberseite 54 der Rückschicht 56 der Windel 50 an. Hierbei gehen die mechanisch wirkenden Verschlusselemente 28 eine lösbar haftende Verbindung mit der textil anmutenden Oberseite 54 ein und halten das Befestigungselement 2 in seiner Z-förmig gefalteten Konfiguration 35. Insbesondere wird verhindert, dass sich der zweite Abschnitt 8 mit dem Bereich 26 von der Oberseite 54 löst. Auf diese Weise ist eine Verbesserung der Handhabbarkeit des Hygieneartikels nach dem Anfügen der Befestigungselemente 2 erreicht, und diese bleiben in ihrer Montageposition. Die Windel 50 kann in der Herstellungsmaschine gehandhabt, insbesondere gefaltet, gestapelt und einer Verpackung zugeführt werden.

In Figur 4 links ist der entfaltete Zustand der Befestigungselemente 2 dargestellt. Hierfür ergreift ein Benutzer den Anfaßbereich 44 und mit der anderen Hand umfaßt er einen Längsrandbereich 52 der Windel 50 und zieht die beiden Komponenten auseinander. Hierdurch wird die lösbar haftende Verbindung der Verschlusselemente 28 an der textil anmutenden Oberseite 54 gelöst, und die Befestigungselemente 2 werden unter Entfaltung der Z-förmig gefalteten Konfiguration 35 in die in Figur 4 links dargestellte Gestalt gebracht. Die Befestigungselemente können nun zum Anlegen der Windel 50 an einen Benutzer auf einen Auftreffbereich 55 in Form eines Schlaufenmaterials im Vorderbereich der Windel 50 gebracht und dort befestigt werden.

Zur weiteren Fixierung der Befestigungselemente 2 in Z-förmig gefalteter Konfiguration 35 ist es denkbar, diskrete Fixierpunkte, etwa Schweißpunkte 51 vorzusehen, welche die Z-förmig übereinander gefalteten Lagen des Befestigungselements 2 lösbar miteinander verbinden. Es hat sich aber gezeigt, dass eine den Anforderungen genügende Fixierung der Z-förmig gefalteten Konfiguration 35 dadurch erreicht werden kann, dass beim Abtrennen der die jeweiligen Befestigungselemente bildenden Längsabschnitte von einer Endlosbahn Fasern der übereinander angeordneten, Z-förmig gefalteten Lagen in die jeweils darunter liegende Lage hinein gezogen werden, so dass eine Art Vernadelungseffekt entsteht, der die Z-förmige Konfiguration 35 lösbar auf sich selbst fixiert.

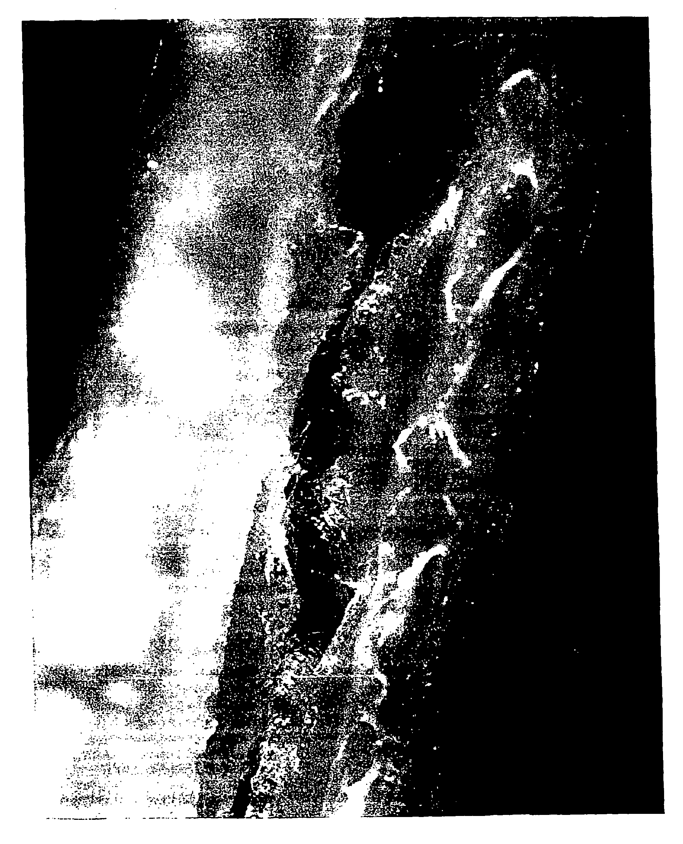

Figur 5 zeigt in schematischer Andeutung eine Schneidvorrichtung zum Abtrennen von Längsabschnitten 90 von einer Endlosbahn 92, welche zur Bildung der Befestigungselemente schon die Z-förmig gefaltete Konfiguration aufweist. Die Schneidvorrichtung umfasst eine Ambosswalze 94, über welche die Endlosbahn 92 geführt ist und eine Messerwalze 96 mit einem unter Vorspannung stehenden und schwimmend gelagerten oder in sich nachgiebigen Schneidmesser 98 mit einer Schneidkante 100. Zur Ausführung des Schnitts ist die Scheidwalze 96 derart gegenüber der Ambosswalze 94 positioniert, dass die Schnittkante 100 des Schneidmessers 98 die Oberfläche der Ambosswalze 94 berührt und geringfügig ausweichen kann. Die Endlosbahn 92 wird also über einen zwar verhältnismäßig kurzen Umfangsbereich von der Schneidkante 100 des Schneidmessers 98 beaufschlagt. Hierdurch werden im Unterschied zu einem bloßen Abscheren Fasern der übereinander angeordneten Lagen bei der Ausführung dieses "Quetschschnittes" aus der einen Lage ausgelenkt und in die darunterliegende Lage hineingezogen, hineingequetscht oder hineingewirkt, so dass im abgetrennten Längsabschnitt 90 die Z-förmig übereinander gefalteten Lagen zumindest geringfügig miteinander verbunden sind und so ein unbeabsichtigtes Auffalten vermieden wird.

Aus Figur 4 ist ferner eine Aufteilung in Vorderbereich 60, Rückbereich 62 und dazwischen liegender Schrittbereich 64 ersichtlich. Der Schrittbereich 64 ist dabei bestimmt als derjenige Bereich, welcher Beinausschnitte 66 zur Bildung von Beinöffnungen im angelegten Zustand der Windel umfaßt. Bei Windeln, bei denen keine Beinausschnitte vorgesehen sind, deren Breite oder Erstreckung in Querrichtung 14 sich nicht oder nur unwesentlich ändert, ist der Schrittbereich als derjenige Bereich definiert, auf dessen Längserstreckung die die Beinöffnungen begrenzenden Längsrandabschnitte verlaufen. Vorderbereich 60 und Rückbereich 62 schließen sich dann an den bezüglich einer Quermittellinie 68 symmetrischen Schrittbereich 64 in Längsrichtung 12 an.

Wie in der Figur 4 dargestellt ist, ist in einem Längsendbereich 70 des Rückbereichs 62 beidseits in Querrichtung 14 außen je eine Materialaussparung 72, 74 vorgesehen. Die Materialaussparung 72, 74 ist von einem bogenförmigen Rand 76 bzw. 78 begrenzt, der von dem Längsrand 58 zunächst schräg und dann im wesentlichen in Querrichtung 14 nach innen verläuft und schließlich bis in die Längsrichtung 12 umgelenkt wird und im wesentlichen in der Längsrichtung 12 dann im wesentlichen senkrecht auf einen den Rückbereich 62 begrenzenden ersten Querrand 80 trifft. Durch die Aussparungen 72, 74 ist die Erstreckung Lₐ im Bereich des Längsseitenrands 50 geringer als die Längserstreckung Lᵢ im Bereich zwischen den Aussparungen 72, 74. Hierdurch wird der Eindruck einer schlanken Windel erzeugt.

Es erweist sich als vorteilhaft, dass die Erstreckung der Befestigungselemente 2 in der Längsrichtung 12 etwa 50% der Längserstreckung des gesamten Rückbereichs 62 beträgt, so dass eine sehr gleichmäßige Einleitung von beim Schließen der Windel auftretenden Zugkräften in das die Außenseite bildende Material der Windel erreicht wird.

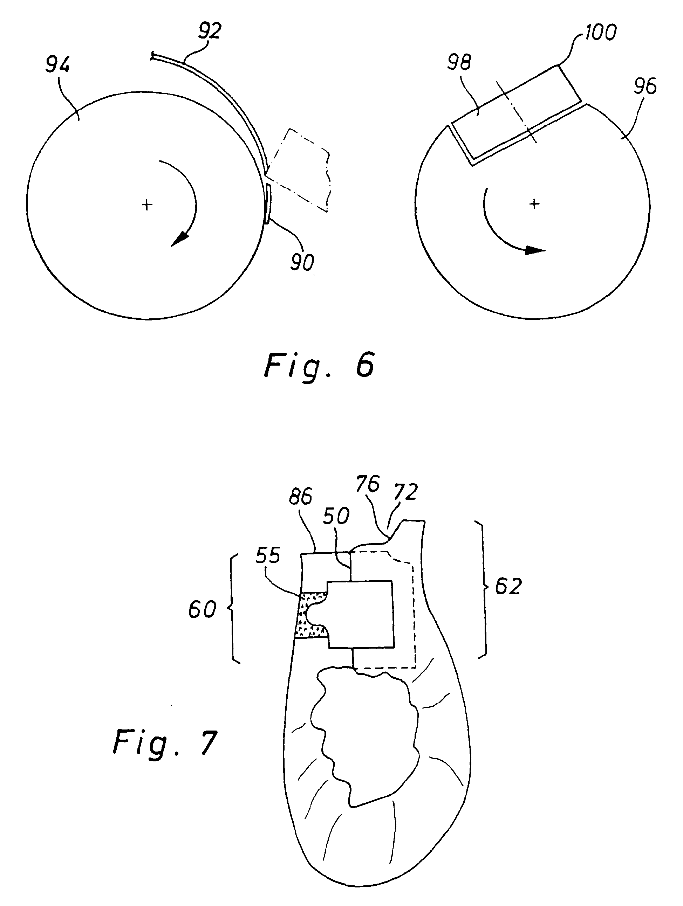

Des weiteren zeigt Figur 4, dass die Längserstreckung des Auftreffbereichs 55 (angedeutet durch den Pfeil 82) der Längserstreckung der Verschlußelemente 28 des Befestigungselements 2 (angedeutet durch den Pfeil 84) im wesentlichen entspricht. Auf diese Weise wird eine korrekte Positionierung der Befestigungselemente 2 im Auftreffbereich 55 und damit ein korrekter Sitz der Windel erzwungen. Es ergibt sich dann die in Figur 7 von der Seite dargestellte Paßform. Man erkennt, dass der die Aussparungen 72, 74 begrenzende Rand 76, 78 im Übergang zum Längsseitenrand 50 des Rückbereichs 62 in gleicher Höhe angeordnet ist wie ein zweiter Querrand 86, welcher ein Längsende des Vorderbereichs 60 der Windel begrenzt. Hierdurch ist eine optimale Paßform der Windel erreicht. Im dargestellten Fall der Figuren 4 unf 7 ist im Vorderbereich 60 eine den Aussparungen 72, 74 entsprechende jedoch weniger ausgeprägte Aussparung 88 vorgesehen. Dies ist jedoch nicht zwingend erforderlich.

## Patentansprüche

1. Hygieneartikel, insbesondere Windel auch für die Inkontinentenversorgung, mit einer flüssigkeitsdurchlässigen Deckschicht, einer flüssigkeitsundurchlässigen Rückschicht und einem dazwischen angeordneten Saugkörper, und mit Befestigungselementen (2) zum lösbaren Schließen des Hygieneartikels im an einen Benutzer angelegten Zustand, wobei der Hygieneartikel eine erste Längsrichtung (12) und eine zweite Querrichtung (14) aufweist, wobei die Befestigungselemente (2) jeweils ein Fixiermittel (27) tragen und die Fixiermittel (27) mit einem Auftreffbereich (55) des Hygieneartikels zusammenwirken, und wobei das jeweilige Befestigungselement (2) einen ersten Bereich (22) aufweist, mit dem es an den Hygieneartikel angefügt ist, und einen zweiten Bereich (26), der in Querrichtung (14) von dem ersten Bereich (22) beabstandet angeordnet ist und in dem jeweils das Fixiermittel (27) vorgesehen ist, wobei das jeweilige Befestigungselement mit in der Längsrichtung (12) verlaufenden Falzlinien (32, 34) z-förmig gefaltet ist,
**dadurch gekennzeichnet,**
**dass** der das Fixiermittel (28) aufweisende zweite Bereich (26) in Querrichtung (14) außerhalb der z-förmig gefalteten Konfiguration (35) liegt.

2. Hygieneartikel nach Anspruch 1, **dadurch gekennzeichnet, dass** das jeweilige Befestigungselement (2) mit einem außerhalb der z-förmig gefalteten Konfiguration (35) liegenden Bereich mit einer Oberseite (54) des Hygieneartikels eine lösbare Halteverbindung eingeht.

3. Hygieneartikel nach Anspruch 1 oder 2, **dadurch gekennzeichnet, dass** das jeweilige Fixiermittel (27) durch mechanisch wirkende Verschlußelemente (28) gebildet ist.

4. Hygieneartikel nach Anspruch 2 oder 3, **dadurch gekennzeichnet, dass** die lösbare Halteverbindung von dem Fixiermittel (27) im zweiten Bereich (26) selbst gebildet ist.

5. Hygieneartikel nach einem der vorstehenden Ansprüche, **dadurch gekennzeichnet, dass** die Befestigungselemente (2) an eine körperabgewandte Oberseite des Hygieneartikels angefügt sind.

6. Hygieneartikel nach Anspruch 5, **dadurch gekennzeichnet, dass** die Oberseite eine textil anmutende Oberfläche (54) aufweist, die von einem Folien/Vlies-Laminat gebildet sein kann, welches die flüssigkeitsundurchlässige Rückschicht (56) umfasst.

7. Hygieneartikel nach einem oder mehreren der vorstehenden Ansprüche, **dadurch gekennzeichnet, dass** die z-förmig gefalteten Befestigungselemente (2) in Querrichtung (14) derart bezüglich eines Längsrands (58) des Hygieneartikels positioniert sind, dass der das jeweilige Fixiermittel (27) tragende zweite Bereich (26) wenigstens nahezu vollständig innerhalb des Längsrands (58) angeordnet ist.

8. Hygieneartikel nach einem oder mehreren der vorstehenden Ansprüche, **dadurch gekennzeichnet, dass** in Querrichtung (14) außerhalb des zweiten Bereichs (26) des jeweiligen Befestigungselements (2) und auf der von der gefalteten Konfiguration (35) abgewandten Seite des zweiten Bereichs (26) ein zum Ergreifen mit einem Finger des Benutzers dienender Anfassbereich (44) vorgesehen ist.

9. Hygieneartikel nach Anspruch 8, **dadurch gekennzeichnet, dass** das jeweilige Befestigungselement (2) mit seinem Anfassbereich (44) in Querrichtung (14) über einen Längsrand (58) des Hygieneartikels übersteht.

10. Hygieneartikel nach einem oder mehreren der vorstehenden Ansprüche, **dadurch gekennzeichnet, dass** das jeweilige Befestigungselement (2) einen ersten Abschnitt (4) einer ersten Trägerschicht (6) und einen zweiten Abschnitt (8) einer zweiten Trägerschicht (10) aufweist, der in Querrichtung (14) neben dem ersten Abschnitt (4) angeordnet und mit diesem verbunden ist, und dass der erste Abschnitt (4) den ersten Bereich (22) und der zweite Abschnitt (8) den zweiten Bereich (26) aufweist.

11. Hygieneartikel nach Anspruch 10, **dadurch gekennzeichnet, dass** eine in der Längsrichtung (12) verlaufende Falzlinie (34) des Befestigungselements (2) wenigstens abschnittsweise entlang eines Materialübergangs zwischen erstem und zweitem Abschnitt (4, 8) verläuft.

12. Hygieneartikel nach Anspruch 11, **dadurch gekennzeichnet, dass** die Falzlinie (34) unmittelbar entlang eines Längsrands (38) des zweiten Abschnitts (8) verläuft.

13. Hygieneartikel nach einem der Ansprüche 10, 11 oder 12, **dadurch gekennzeichnet, dass** der erste und der zweite Abschnitt (4, 8) des Befestigungselements (2) in Querrichtung (14) überlappt sind und im Überlappungsbereich (16) zusammengefügt sind.

14. Hygieneartikel nach einem oder mehreren der vorstehenden Ansprüche, **dadurch gekennzeichnet, dass** eine in der Längsrichtung (12) verlaufende Falzlinie (32) des Befestigungselements (2) unmittelbar entlang eines Längsrands (36) des ersten Bereichs (22) verläuft.

15. Hygieneartikel nach Anspruch 14, **dadurch gekennzeichnet, dass** die Falzlinie (32) unmittelbar entlang eines Längsrands (36) einer Kleberbeschichtung (24) im ersten Bereich (22) verläuft.

16. Hygieneartikel nach einem oder mehreren der Ansprüche 10-15, **dadurch gekennzeichnet, dass** die zweite Trägerschicht (10) des Befestigungselements (2) ein im wesentlichen unelastisches Material umfasst und in Querrichtung (14) im wesentlichen nicht dehnbar ausgebildet ist.

17. Hygieneartikel nach einem oder mehreren der Ansprüche 10-16, **dadurch gekennzeichnet, dass** die erste Trägerschicht (6) des Befestigungselements (2) ein zumindest in Querrichtung (14) elastisch dehnbares Material umfasst und in dieser Querrichtung (14) elastisch dehnbar ausgebildet ist.

18. Hygieneartikel nach einem oder mehreren der vorstehenden Ansprüche, **dadurch gekennzeichnet, dass** der erste Bereich (22) klebend ausgebildet ist.

19. Hygieneartikel nach einem oder mehreren der vorstehenden Ansprüche, **dadurch gekennzeichnet, dass** die z-förmig gefaltete Konfiguration (35) des Befestigungselements (2) auf sich selbst fixiert ist.

20. Hygieneartikel nach Anspruch 19, **dadurch gekennzeichnet, dass** die z-förmig gefaltete Konfiguration (35) auf sich selbst fixiert ist durch eine Anzahl von 1-10, vorzugsweise 3-5 diskreten Fixierpunkten (51) mit einer Querschnittsfläche von jeweils weniger als 1 mm².

21. Hygieneartikel nach Anspruch 19 oder 20, **dadurch gekennzeichnet, dass** die z-förmig gefaltete Konfiguration (35) des Befestigungselements (2) **dadurch** auf sich selbst fixiert ist, dass beim Abtrennen von Längsabschnitten in Querrichtung (14) von einer endlosen Bahn am Schnittrand Fasern der z-förmig übereinander gefalteten Lagen in die darunter angeordnete Lage hineingewirkt, hineingequetscht oder hineingearbeitet sind.

22. Hygieneartikel nach einem oder mehreren der Ansprüche 8-21, **dadurch gekennzeichnet, dass** ein Anfassbereich (44) des Befestigungselements (2) von einer nicht haftenden Trägerschicht (10) selbst gebildet ist.

23. Hygieneartikel nach einem oder mehreren der vorstehenden Ansprüche, **dadurch gekennzeichnet, dass** die Erstreckung des Befestigungselements (2) in der Längsrichtung 2-10 cm, insbesondere 5 - 7 cm beträgt.

24. Hygieneartikel nach einem oder mehreren der vorstehenden Ansprüche 10 - 23, **dadurch gekennzeichnet, dass** die Erstreckung des ersten Abschnitts (4) des Befestigungselements (2) in der Querrichtung (14) in einem elastisch ausgebildeten Bereich zwischen dem Längsrand (6) des ersten Bereichs (22) und dem Materialübergang zum zweiten Abschnitt (8) 0,5 - 4,5 cm, insbesondere 2 - 3 cm beträgt.

25. Hygieneartikel nach einem oder mehreren der vorstehenden Ansprüche, **dadurch gekennzeichnet, dass** die Erstreckung des das Fixiermittel (27) aufweisenden zweiten Bereichs (26) des Befestigungselements (2) in der Querrichtung (14) 0,5 - 2,1 cm, insbesondere 1 - 1,5 cm beträgt.

26. Hygieneartikel nach einem oder mehreren der vorstehenden Ansprüche, **dadurch gekennzeichnet, dass** die Erstreckung des Anfassbereichs (44) des Befestigungselements (2) in der Querrichtung (14) 0,5 - 2,0 cm, insbesondere 0,7 - 1,2 cm beträgt.

27. Hygieneartikel nach einem oder mehreren der vorstehenden Ansprüche, **dadurch gekennzeichnet, dass** die Erstreckung des insbesondere klebend ausgebildeten ersten Bereichs (22) des Befestigungselements (2) in Querrichtung (14) im wesentlichen der Breite der z-förmig gefalteten Konfiguration (35) entspricht.

28. Hygieneartikel nach einem oder mehreren der vorstehenden Ansprüche, **dadurch gekennzeichnet, dass** der erste Bereich (22) und der zweite Bereich (26) auf derselben Seite (20) einer endlosen Bahn vorgesehen sind.

## Claims

1. Hygiene article, in respective nappies also for incontinence provision, with a fluid-impervious top sheet, a fluid-impervious back sheet and an absorbent body disposed therebetween, and with fastening elements (2) for the releasable closure of the hygiene article when applied to a user, the hygiene article having a first longitudinal direction (12) and a second transverse direction (14), the fastening elements (2) each carrying a securing means (27), and the securing means cooperating with a contact region (55) of the hygiene article, and wherein the respective fastening element (2) has a first region (22) with which it is attached to the hygiene article and a second region (26), located spaced apart in the transverse direction (14) from the first region (22) and in which the respective securing means (27) is provided in each case, the respective fastening element being folded in a Z-shape with fold lines (32, 34) running in the longitudinal direction (12), **characterised in that** the second region (26) containing the securing means (28) lies outside the Z-shape folded configuration (35).

2. Hygiene article according to claim 1, **characterised in that** the respective fastening element (2) with a region lying outside the Z-shape folded configuration (35) forms a releasably retentive connection with a top side (54) of the hygiene article.

3. Hygiene article according to either claim 1 or claim 2, **characterised in that** the respective securing means (27) is formed by mechanically acting closures (28).

4. Hygiene article according to either claim 2 or claim 3, **characterised in that** the releasably retentive connection is formed by the securing means (27) in the second region (26).

5. Hygiene article according to any one of the preceding claims, **characterised in that** the fastening elements (2) are attached to a top side of the hygiene article facing away from the body.

6. Hygiene article according to claim 5, **characterised in that** the top side has a textile-like surface (54) formed by a film/nonwoven laminate which comprises the fluid-impervious back sheet (56).

7. Hygiene article according to one or more of the preceding claims, **characterised in that** the Z-shape folded fastening elements (2) are positioned in the transverse direction (14) with respect to a longitudinal edge (58) of the hygiene article such that the second region (27), carrying the respective fastening means (27), is configured at least almost entirely inside the longitudinal edge (58).

8. Hygiene article according to one or more of the preceding claims, **characterised in that** a pull tab (44) to be grasped with a user's finger is provided in the transverse direction (14) outside the second region (26) of the respective fastening element (2) and on the side of the second region (20) facing away from the folded configuration (35).

9. Hygiene article according to claim 8, **characterised in that** the pull tab (44) of the respective fastening element (2) projects beyond a longitudinal edge (58) of the hygiene article in the transverse direction (14).

10. Hygiene article according to one or more of the preceding claims, **characterised in that** the respective fastening element (2) has a first portion (4) of a first supporting layer (6) and a of a second portion (8) of a second supporting layer (10), which is disposed next to the first portion (4) in the transverse direction (14) and connected thereto, and **in that** the first portion (4) has the first region (22) and the second portion (8) has the second region (26).

11. Hygiene article according to claim 10, **characterised in that** a fold line (34) of the fastening element (2) running in the longitudinal direction (12) runs at least in portions along a material transition between the first and second portion (4, 8).

12. Hygiene article according to claim 11, **characterised in that** the fold line (34) runs immediately along a longitudinal edge (38) of the second portion (8).

13. Hygiene article according to any one of claims 10, 11 or 12, **characterised in that** the first and the second portion (4, 8) of the fastening element (2) are overlapped in the transverse direction (14) and are joined in the overlap area (16).

14. Hygiene article according to one or more of the preceding claims, **characterised in that** a fold line (32) of the fastening element (2) running in the longitudinal direction (12) runs immediately along a longitudinal edge (36) of the first region (22).

15. Hygiene article according to claim 14, **characterised in that** the fold line (32) runs immediately along a longitudinal edge (36) of an adhesive coating (24) in the first region (22).

16. Hygiene article according to one or more of the claims 10 to 15, **characterised in that** the second supporting layer (10) of the fastening element (2) comprises a substantially unresilient material and is configured substantially non-extensibly in the transverse direction (14).

17. Hygiene article according to one or more of claims 10 to 16, **characterised in that** the first supporting layer (6) of the fastening element (2) comprises a material resiliently extensible in at least the transverse direction (14) and is resiliently extensible in this transverse direction (14).

18. Hygiene article according to one or more of the preceding claims, **characterised in that** the first region (22) is configured adhesively.

19. Hygiene article according to one or more of the preceding claims, **characterised in that** the Z-shape folded configuration (35) of the fastening element (2) is secured to itself.

20. Hygiene article according to claim 19, **characterised in that** the Z-shaped folded configuration (35) is secured to itself by 1 to 10, preferably 3 to 5 discrete fixing points (51) with a cross-sectional area, each of less than 1 mm²

21. Hygiene article according to claim 19 or claim 20, **characterised in that** the Z-shape folded configuration (35) of the fastening element (2) is secured to itself in such a means that when longitudinal sections are separated from a continuous web in the transverse direction (14) fibres at the cut edge of the layers folded over each other in a Z-shape are worked, squeezed or incorporated into the layer disposed thereunder.

22. Hygiene article according to one or more of the claims 8 to 21, **characterised in that** a pull tab (44) of the fastening element (2) is formed by a non-adhesive supporting layer (10).

23. Hygiene article according to one or more of the preceding claims, **characterised in that** the extension of the fastening element (2) measures 2 to 10 cm, in the longitudinal direction, in respective 5 to 7 cm.

24. Hygiene article according to one or more of the preceding claims 10 to 23, **characterised in that** the extension of the first portion (4) of the fastening element (2) in a resiliently configured region between the longitudinal edge (6) of the first region (22) and the material transition with the second portion (8) measures 0.5 to 4.5 cm, in respective 2 to 3 cm, in the transverse direction (14).

25. Hygiene article according to one or more of the preceding claims, **characterised in that** the extension of the second region (26) of the fastening element (2) having the securing means (27) measures 0.5 to 2.1 cm, in respective 1 to 1.5 cm, in the transverse direction (14).

26. Hygiene article according to one or more of the preceding claims, **characterised in** the extension of the pull tab (44) of the fastening element (2) measures 0.5 to 2.0 cm, in respective 0.7 to 1.2 cm, in the transverse direction (14).

27. Hygiene article according to one of more of the preceding claims, **characterised in that** the extension of the specifically adhesively configured first region (22) of the fastening element (2) in the transverse direction (14) substantially corresponds to the width of the Z-shape folded configuration (35).

28. Hygiene article according to one or more of the preceding claims, **characterised in that** the first region (22) and the second region (26) are provided on the same side (20) of a continuous web.

## Revendications

1. Article d'hygiène, en particulier couche également destinée aux personnes incontinentes, comprenant une couche de surface perméable aux liquides, une couche arrière imperméable aux liquides et un corps absorbant agencé entre les deux et comprenant des éléments de fixation (2) pour fermer de manière détachable l'article d'hygiène lorsqu'il est placé sur un utilisateur, l'article d'hygiène présentant un premier sens longitudinal (12) et un deuxième sens transversal (14), les éléments de fixation (2) portant chacun un moyen de fixation (27) et les moyens de fixation (27) coopérant avec une zone de contact (55) de l'article d'hygiène et chaque élément de fixation (2) présentant une première zone (22) par laquelle il est joint à l'article d'hygiène et une deuxième zone (26) qui est agencée à distance de la première zone (22) dans le sens transversal (14) et dans laquelle le moyen de fixation (27) est prévu, chaque élément de fixation étant plié en forme de z avec des lignes de pliage (32, 34) s'étendant dans le sens longitudinal (12), **caractérisé en ce que** la deuxième zone (26) présentant le moyen de fixation (27) est située dans le sens transversal (14) en dehors de la configuration pliée en forme de z (35).

2. Article d'hygiène selon la revendication 1, **caractérisé en ce que** chaque élément de fixation (2) avec une zone située en dehors de la configuration pliée en forme de z (35) forme un assemblage de maintien détachable avec une face supérieure (54) de l'article d'hygiène.

3. Article d'hygiène selon la revendication 1 ou 2, **caractérisé en ce que** le moyen de fixation (27) est formé par des éléments de fermeture à action mécanique (28).

4. Article d'hygiène selon la revendication 2 ou 3, **caractérisé en ce que** l'assemblage de maintien détachable est lui-même formé par le moyen de fixation (27) dans la deuxième zone (26).

5. Article d'hygiène selon l'une des revendications précédentes, **caractérisé en ce que** les éléments de fixation (2) sont joints à une face supérieure opposée au corps de l'article d'hygiène.

6. Article d'hygiène selon la revendication 5, **caractérisé en ce que** la face supérieure présente une surface donnant l'impression de textile (54) qui peut être formée par un stratifié film/non tissé qui comprend la couche arrière imperméable aux liquides (56).

7. Article d'hygiène selon l'une ou plusieurs des revendications précédentes, **caractérisé en ce que** les éléments de fixation (2) pliés en forme de z sont positionnés dans le sens transversal (14) par rapport à un bord longitudinal (58) de l'article d'hygiène de telle manière que la deuxième zone (26) portant le moyen de fixation (27) est agencée au moins presque entièrement à l'intérieur du bord longitudinal (58).

8. Article d'hygiène selon l'une ou plusieurs des revendications précédentes, **caractérisé en ce qu'**il est prévu, dans le sens transversal (14) en dehors de la deuxième zone (26) de chaque élément de fixation (2) et du côté de la deuxième zone (26) opposé à la configuration pliée (35), une zone de prise (44) destinée à être saisie par un doigt de l'utilisateur.

9. Article d'hygiène selon la revendication 8, **caractérisé en ce que** chaque élément de fixation (2) dépasse dans le sens transversal (14), avec sa zone de prise (44), d'un bord longitudinal (58) de l'article d'hygiène.

10. Article d'hygiène selon l'une ou plusieurs des revendications précédentes, **caractérisé en ce que** chaque élément de fixation (2) présente un premier segment (4) d'une première couche porteuse (6) et un deuxième segment (8) d'une deuxième couche porteuse (10) qui est agencé dans le sens transversal (14) à côté du premier segment (4) et est relié à celui-ci, et **en ce que** le premier segment (4) présente la première zone (22) et le deuxième segment (8) la deuxième zone (26).

11. Article d'hygiène selon la revendication 10, **caractérisé en ce qu'**une ligne de pliage (34) de l'élément de fixation (2), s'étendant dans le sens longitudinal (12), s'étend au moins par segments le long d'un passage de matériau entre le premier et le deuxième segment (4, 8).

12. Article d'hygiène selon la revendication 11, **caractérisé en ce que** la ligne de pliage (34) s'étend directement le long d'un bord longitudinal (38) du deuxième segment (8).

13. Article d'hygiène selon l'une des revendications 10, 11 ou 12, **caractérisé en ce que** le premier et le second segments (4, 8) de l'élément de fixation (2) se chevauchent dans le sens transversal (14) et sont assemblés dans la zone de chevauchement (16).

14. Article d'hygiène selon l'une ou plusieurs des revendications précédentes, **caractérisé en ce qu'**une ligne de pliage (32) de l'élément de fixation (2), s'étendant dans le sens longitudinal (12), s'étend directement le long d'un bord longitudinal (36) de la première zone (22).

15. Article d'hygiène selon la revendication 14, **caractérisé en ce que** la ligne de pliage (32) s'étend directement le long d'un bord longitudinal (36) d'un revêtement adhésif (24) dans la première zone (22).

16. Article d'hygiène selon l'une ou plusieurs des revendications 10 à 15, **caractérisé en ce que** la deuxième couche porteuse (10) de l'élément de fixation (2) comprend un matériau essentiellement non élastique et est conçue essentiellement non extensible dans le sens transversal (14).

17. Article d'hygiène selon l'une ou plusieurs des revendications 10 à 16, **caractérisé en ce que** la première couche porteuse (6) de l'élément de fixation (2) comprend un matériau extensible élastiquement au moins dans le sens transversal (14) et est formée extensible élastiquement dans ce sens transversal (14).

18. Article d'hygiène selon l'une ou plusieurs des revendications précédentes, **caractérisé en ce que** la première zone (22) est conçue adhésive.

19. Article d'hygiène selon l'une ou plusieurs des revendications précédentes, **caractérisé en ce que** la configuration pliée en forme de z (35) de l'élément de fixation (2) est fixée sur elle-même.

20. Article d'hygiène selon la revendication 19, **caractérisé en ce que** la configuration pliée en forme de z (35) est fixée sur elle-même par un nombre de points de fixation discrets allant 1 à 10, de préférence de 3 à 5 (51), ayant chacun une aire de section transversale inférieure à 1 mm².

21. Article d'hygiène selon la revendication 19 ou 20, **caractérisé en ce que** la configuration pliée en forme de z (35) de l'élément de fixation (2) est fixée sur elle-même **en ce que**, lors de la séparation de segments longitudinaux d'avec une bande sans fin dans le sens transversal (14) au bord de coupe, des fibres des couches pliées les unes sur les autres en forme de z sont enchevêtrées, écrasées ou entremêlées dans la couche située en-dessous.

22. Article d'hygiène selon l'une ou plusieurs des revendications 8 à 21, **caractérisé en ce qu'**une zone de prise (44) de l'élément de fixation (2) est elle-même formée par une couche porteuse (10) non adhésive.

23. Article d'hygiène selon l'une ou plusieurs des revendications précédentes, **caractérisé en ce que** l'étendue de l'élément de fixation (2) dans le sens longitudinal est comprise entre 2 et 10 cm, en particulier entre 5 et 7 cm.

24. Article d'hygiène selon l'une ou plusieurs des revendications 10 à 23, **caractérisé en ce que** l'étendue du premier segment (4) de l'élément de fixation (2) dans le sens transversal (14) dans une zone réalisée élastique entre le bord longitudinal (6) de la première zone (22) et le passage de matériau au deuxième segment (8) est comprise entre 0,5 et 4,5 cm, en particulier entre 2 et 3 cm.

25. Article d'hygiène selon l'une ou plusieurs des revendications précédentes, **caractérisé en ce que** l'étendue de la deuxième zone (26) de l'élément de fixation (2) présentant le moyen de fixation (27) dans le sens transversal (14) est comprise entre 0,5 et 2,1 cm, en particulier entre 1 et 1,5 cm.

26. Article d'hygiène selon l'une ou plusieurs des revendications précédentes, **caractérisé en ce que** l'étendue de la zone de prise (44) de l'élément de fixation (2) dans le sens transversal (14) est comprise entre 0,5 et 2 cm, en particulier entre 0,7 et 1,2 cm.

27. Article d'hygiène selon l'une ou plusieurs des revendications précédentes, **caractérisé en ce que** l'étendue de la première zone conçue en particulier adhésive (22) de l'élément de fixation (2) dans le sens transversal (14) correspond sensiblement à la largeur de la configuration pliée en forme de z (35).

28. Article d'hygiène selon l'une ou plusieurs des revendications précédentes, **caractérisé en ce que** la première zone (22) et la deuxième zone (26) sont prévues du même côté (20) d'une bande sans fin.
